# EUROPEAN PATENT SPECIFICATION

(11) **EP 3 585 792 B1**
(45) Date of publication and mention of the grant of the patent: **21.06.2023**
(21) Application number: 18707677.3
(22) Date of filing: 22.02.2018
(51) Int. Cl.: C07H 17/02, C07H 15/203, C07B 59/00, A61K 51/04

(54) **RADIOLABELED BETA-GALACTOSIDASE SUBSTRATE FOR PET IMAGING OF SENESCENCE**
RADIOAKTIV MARKIERTES BETA-GALAKTOSIDASE-SUBSTRAT ZUR PET-BILDGEBUNG VON SENESZENZ
SUBSTRAT DE BÊTA-GALACTOSIDASE RADIOMARQUÉ POUR L'IMAGERIE PET DE LA SÉNESCENCE

(30) Priority: 22.02.2017 DE 102017103600
(43) Date of publication of application: 01.01.2020
(73) Proprietor: Eberhard Karls Universität Tübingen Medizinische Fakultät, 72074 Tübingen (DE)
(72) Inventor: COTTON, Jonathan, 72070 Tuebingen (DE); KUEHN, Anna, 78609 Tuningen (DE); MAURER, Andreas, 72076 Tuebingen (DE); PICHLER, Bernd, 85298 Scheyern (DE); SCHULZE-OSTHOFF, Klaus, 72070 Tuebingen (DE); FUCHS, Kerstin, 71088 Holzgerlingen (DE); KRUEGER, Marcel André, 72764 Reutlingen (DE); ZENDER, Lars, 72108 Rottenburg - Wendelsheim (DE)
(74) Representative: Witte, Weller & Partner Patentanwälte mbB
(86) International application number: PCT/EP2018/054356
(87) International publication number: WO 2018/153966

(56) References cited:
- WO-A2-2012/174285
- RUOHO A E ET AL: "Development of Novel Photoaffinity Ligands for the beta-Adrenergic Receptor", NEUROPROTOCOLS, ACADEMIC PRESS, ORLANDO, FL, US, vol. 4, no. 1, 1 February 1994 (1994-02-01), pages 50-65, XP024875349, ISSN: 1058-6741, DOI: 10.1006/NCMN.1994.1007 [retrieved on 1994-02-01]
- CARA L. FERREIRA ET AL: "Carbohydrate-Appended 3-Hydroxy-4-pyridinone Complexes of the [M(CO) 3 ] + Core (M = Re, 99m Tc, 186 Re)", BIOCONJUGATE CHEMISTRY, vol. 17, no. 5, 1 September 2006 (2006-09-01), pages 1321-1329, XP055474081, US ISSN: 1043-1802, DOI: 10.1021/bc060085e
- SOFIE CELEN ET AL: "Synthesis and Evaluation of 18 F- and 11 C-Labeled Phenyl-Galactopyranosides as Potential Probes for in Vivo Visualization of LacZ Gene Expression using Positron Emission Tomography", BIOCONJUGATE CHEMISTRY, vol. 19, no. 2, 1 February 2008 (2008-02-01), pages 441-449, XP055053437, ISSN: 1043-1802, DOI: 10.1021/bc700216d

## Description

The present invention relates to compounds useful for visualizing cell senescence *in vitro* and *in vivo,* the preparation of said compounds and their use. In particular, the present invention pertains to novel hexose and particularly galactose derivatives which are useful as senescence tracers *in vitro* and *in vivo.*

Cell senescence is the biological process by which cells exit the growth cycle. Cell senescence was first characterized in fibroblasts, which could only be subjected to a limited number of passages, before growth became permanently arrested. This phenomenon is named the Hayflick limit and serves to explain the physiological course of aging. It is accompanied by distinct change in metabolic pathways (Roninson, E.B., Tumor Cell Senescence in Cancer Treatment, Cancer Res., 2003, 63:2705-2715).

Senescent cells exhibit a senescence associated secretory phenotype, containing pro-inflammatory cytokines and growth factors. In certain instances, the removal of senescent tissues can convey great benefits to a patient. Senescence is recognized to play an important role in cancer treatment and therapy resistance. Treatment-associated senescence marks as stable clinical end point, and thus can be a measurement of chemotherapeutic success. The detection of senescence cells might also offer diagnostic opportunities for detecting pre-neoplastic lesions (Roninson, E.B., Tumor Cell Senescence in Cancer Treatment, Cancer Res., 2003, 63:2705-2715;and Campisi, J. and d'Adda di Fagagna, F., Cellular Senescence: when bad things happen to good cells, Nature Reviews Molecular Cell Biology, 2007, 8:729-740).

The most widely used surrogate marker for senescence cells is the senescence-associated beta-galactosidase (Roninson, E.B., Tumor Cell Senescence in Cancer Treatment, Cancer Res., 2003, 63:2705-2715). Such beta-galactosidase substrates have, however, the drawback that they merely may be used ex vivo and *in vitro* for following beta-galactosidase expression. Sofie Gelen et al: "Synthesis and Evaluation of 18F- and 11C-Labeled Phenyl-Galactopyranosides as Potential Probes for in Vivo Visualization of LacZ Gene Expression using Positron Emission Tomography", BIOCONJUGATE CHEMISTRY, vol. 19, no. 2, 1 February 2008, pages 441-449 discloses inter alia the compound 3-(2'-[¹⁸F]fluoroethoxy)-2-nitrophenyl beta-D-galactopyranoside.
WO 2012/174285 A2 discloses radiolabeled tracers for binding to sodium/glucose co-transporters (SGLTs) and their synthesis.

The present invention seeks to overcome the problems associated with prior art compounds.

There exists therefore an unmet need for further compounds that can act as tracers for cell senescence which may be used *in vitro* and *in vivo* at the same time. There is also a need in developing improved methods in the treatment of disorders related to cell senescence, such as cancer. A further objective of the present invention resides in alternative methods for selectively eliminating senescent cells *in vivo.* Still a further objective of the present invention is the provision of alternative means for determining the efficiency of cancer treatment.

The present invention is based on the finding that hexose derivatives, preferably galactoside derivatives and more preferably beta galactoside derivatives, carrying radioactive labels accumulate in senescent cells and can be used to reliably mark and detect said senescent cells both *in vitro* and *in vivo. In vivo* detection of senescent cells offers in turn improved access to such cells by surgery or therapy.

The present invention therefore provides a compound of the formula:

G - S - L,

wherein
G is 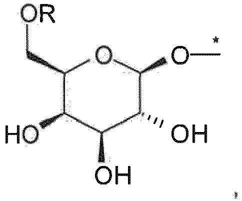 wherein R is H,
   and wherein * represents the binding site between G and S,
S is selected from the group consisting of 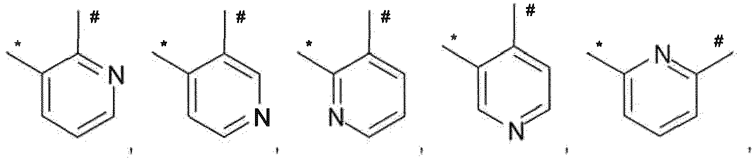 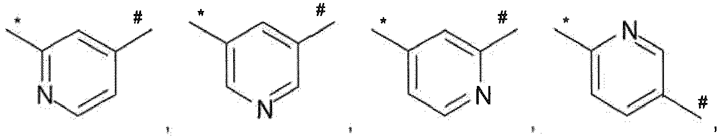 and 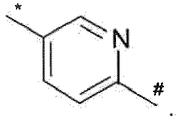 wherein # represents the binding site between S and L, and
L is 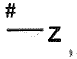
   wherein Z is a radioactive detectable label, a radioactive therapeutic residue;
   or a salt thereof, wherein the radioactive detectable label is selected from the group consisting of ¹¹C, ⁴⁰K, ¹³N, ¹⁵O, ¹⁸F, ⁷⁵Br, ⁷⁶Br, ⁸²Rb, ⁶⁸Ga, ⁶⁴Cu, ⁶²Cu, ⁸⁹Zr, ¹²³I, ¹²⁴I, ¹²⁵I, ¹³¹I, ²¹⁰At, ²¹¹At and ¹¹¹In,
   wherein the radioactive therapeutic residue is selected from the group consisting of ³²P, ⁶⁰Co, ⁶⁴Cu, ⁸⁹Sr, ⁹⁰Y, ¹⁷⁷Lu,¹⁸⁶Re and ¹⁵³Sm.

It has been surprisingly found that the present compounds are highly and selectively enriched in senescent cells. Without wishing to be bound by theory, it is assumed that the rather small size of radioactive detectable labels in comparison to conventional non-radioactive labels, which is often based on the detection of bulky enzymes, such as horseradish peroxidase, alkaline phosphatase, or bulky dye compounds, offers improved access of the present compounds to the cells and the beta-galactosidase's binding site. Furthermore, the use of radioactive labels confers the advantage of highly enhanced sensitivity versus conventional labeling strategies. This is in part due to the high tissue penetration of the resulting gamma photons and very high sensitivity of the radio detectors used. This in turn allows for the denoted compounds to be translatable, in vivo, making them superior to nonradioactive alternatives. The present compounds thereby offer the possibility to quantitatively, selectively and sensitively detect senescent cells, particularly tumors, *in vivo.* This highly selective enrichment in senescent cells may be used in diagnosis or treatment of senescence related disorders by employing radioactive detectable labels or radioactive therapeutic residues in the present compounds.

Another advantage resides in the possibility to treat tumors with non-invasive techniques, i.e. by administering the present compounds exhibiting a radioactive therapeutic residue, which are almost exclusively incorporated in tumor tissue. Another advantage of the use of the present compounds resides in that they may be easily adapted to the changing requirements in the clinical field since the present compound may be easily provided with another radioactive detectable label or radioactive therapeutic residue.

The compounds of the invention may, depending on their structure, exist in stereoisomeric forms (enantiomers, diastereomers). The invention therefore also encompasses the enantiomers or diastereomers and respective mixtures thereof. The stereoisomerically uniform constituents can be isolated in a known manner from such mixtures of enantiomers and/or diastereomers.

It is clear that the salts preferred for the purposes of the present invention are pharmaceutically acceptable salts of the compounds of the invention. However, also encompassed are salts which are themselves not suitable for pharmaceutical applications but can be used for example for the isolation or purification of the compounds of the invention.

Salts in general are composed of related numbers of cations and anions so that the product is electrically neutral. It will be appreciated that a respective counterion is subject to the preparation process of the salt and needs not to be expressively mentioned.

Pharmaceutically acceptable salts as well as the preparation thereof are well-known in the art. Types and preparation of such pharmaceutically acceptable salts may be derived from Stahl, P.H. and Wermuth, C.G., Handbook of Pharmaceutical Salts: Properties, Selection and Use, Weinheim/Zürich: Wiley-VCH/VHCA, 2002.

Examples of pharmaceutically acceptable salts of the present compounds include salts of inorganic basis like ammonium salts, alkali metal salts, alkaline earth metal salts, salts of organic basis, or salts with basic amino acids. Also included are inorganic acids or salts with acidic amino acids. Preferred pharmaceutically acceptable salts encompass

It will be appreciated that the compounds of the present invention as well as the salts thereof may be present in form of solvates. In such a case, the present compounds or salts, particularly the pharmaceutically acceptable salts, thereof form in the solid or liquid state a complex by coordination with solvent molecules. Hydrates are a specific form of solvates in which the coordination displays with water.

If the compounds of the present invention may occur in tautomeric forms, the present invention encompasses all tautomeric forms.

The term "C₁-C₅ alkyl" generally refers to branched or straight-chain alkyl, preferably (C₁-C₄)-alkyl, such as in particular methyl, ethyl, propyl, butyl, isopropyl, isobutyl and tert.-butyl.

The term "C₁-C₁₀ cycloalkyl" generally refers to saturated or partially unsaturated monocyclic or polycyclic ring comprising carbon and hydrogen atoms.

The term "C₁-C₁₀ heterocycloalkyl" means a non-aromatic monocyclic or polycyclic ring comprising carbon and hydrogen atoms and at least one heteroatom, preferably, 1 to 4 heteroatoms selected from nitrogen, oxygen, and sulfur. A heterocycloalkyl group can have one or more carbon-carbon double bonds or carbon-heteroatoms double bonds in the ring as long as the ring is not rendered aromatic by their presence. Examples of heterocycloalkyl groups include aziridinyl, pyrrolidinyl, pyrrolidino, piperidinyl, piperidino, piperazinyl, piperazino, morpholinyl, morpholino, thiomorpholinyl, thiomorpholino, tetrahydrofuranyl, tetrahydrothiofuranyl, tetrahydropyranyl, and pyranyl. Preferably, the heterocycloalkyl group is a monocyclic or bicyclic ring, more preferably, a monocyclic ring, wherein the ring comprises from 2 to 6 carbon atoms and from 1 to 3 heteroatoms. More preferably the molecular weight of a heterocycloalkyl residue has a molecular weight of ≤ 200 g/mol, such as ≤ 150 g/mol, ≤ 120 g/mol or ≤ 100 g/mol.

The term "substituted" as used herein refers to one or more residues connected to C₁-C₆ alkyl, C₁-C₁₀ cycloalkyl and C₁-C₁₀ heterocycloalkyl and/or a modification to the carbon chain by introducing one or more heteroatoms, such as N; S or O. A residue may be furthermore selected from the group consisting of halogen, methyl, ethyl or functional groups, such as a hydroxyl group, amino group or carboxyl group. The overall molecular weight of residues connected to particular C₁-C₅ alkyl, C₁-C₁₀ cycloalkyl or C₁-C₁₀ heterocycloalkyl is preferably ≤ 250 g/mol, more preferably ≤ 150 g/mol or ≤ 100 g/mol. Examples of preferred substituted to C₁-C₅ alkyl residues encompass compounds such as CH₂-O-CH₂, C₂H₄-O-CH₂, CH₂-O-CH₂-O-CH₂, CH₂-O-CH₂-O-CH₂, C₂H₄-O-CH₂-O-CH₂, CH₂-O- C₂H₄-O-CH₂, C₂H₄-O-C₂H₄-O-CH₂, C₂H₄-O-CH₂-O- C₂H₄, CH₂-O-CH₂-O-CH₂-O-CH₂, C₂H₄-O-CH₂-O-CH₂-O-CH₂, and CH₂-O- C₂H₄-O-CH₂-O-CH₂.

The term "halogen" or halo refers to fluorine, chlorine, bromine or iodine; preferably fluorine, chlorine or bromine. In some embodiments the term halogen or halo preferably refers to ⁷⁶Br, ⁷⁵Br, ¹⁹F and ¹⁸F.

The term "methyl halogen" refers to a methyl group having a single fluorine, chlorine, bromine or iodine, or two or three halogens which are independently selected from fluorine, chlorine, bromine or iodine. In some embodiments the methyl halogen exhibits preferably ⁷⁶Br, ⁷⁵Br, ¹⁹F and ¹⁸F.

The term "radioactive detectable label" encompasses for instance ¹¹C, ⁴⁰K, ¹³N, ¹⁵O, ¹⁸F, ⁷⁵Br, ⁷⁶Br, ⁸²Rb, ⁶⁸Ga, ⁶⁴Cu, ⁶²Cu, ¹²³I, ¹²⁴I, ¹²⁵I, ¹³¹I, ²¹⁰At, ²¹¹At and ¹¹¹In. Preferred radioactive detectable labels are ¹¹C, ¹⁸F, ⁶⁸Ga, ⁶⁴Cu, and ¹²⁴I. More preferred is ¹⁸F. The radioactive detectable label allows quantitatively and/or qualitatively assessment of senescent cells as well as their location particularly in vivo. Hence, senescent tissue may be properly identified and for instance surgically removed with high selectivity.

Alternatively, a complex containing such an atom in a coordinated form is encompassed. In such a case the complex may be also denominated a chelator coordinating a radioactive detectable label. A (coordination) complex consists of a central atom or ion, which is usually metallic, and a surrounding array of *ligands* or complexing agents. Usually the central atom or ion is the radioactive detectable label. Residue Z therefore encompasses the central atom or ion as well as the surrounding array of *ligands* or complexing agents. Examples of such chelators coordinating a radioactive detectable label are well known to the skilled person. Preferably such a chelator is an amino acid, preferably a proteinogenic/ natural amino acid, such as histidine. Other suitable examples of chelators comprise DOTA, NOTA, NODAGA and desferrioxamine, such as desferrioxamine B.

The term "radioactive therapeutic residue" as used herein refers to an atom, such as ³²P, ⁶⁰Co, ⁸⁹Sr, ¹⁸⁶Re and ¹⁵³Sm. Other examples of radioactive therapeutic residues encompass ¹²⁵I and ¹³¹I, which have been already mentioned as examples for radioactive detectable labels, as well as ⁸⁶Y, ¹¹¹In, ¹⁷⁷Lu and ⁶⁷Cu. Alternatively, a complex containing such an atom in a coordinated form is encompassed. In such a case the complex may be also denominated a chelator coordinating a radioactive therapeutic residue. The use of the present compounds with therapeutic residues in treatment of disorders associated with cell senescence, particularly cancer, combines the advantage of target selectivity with that of being systemic, as with chemotherapy, and it may be used as part of a therapeutic strategy with curative intent or for disease control and palliation.

It will be appreciated that respective radioactive therapeutic residues may be generated and attached to the present compounds in the same manner as radioactive detectable labels. Preferably, therapeutic residue and detectable label are identical permitting diagnosis and treatment of disorders associated with cell senescence, particularly cancer.

In formulae, the group which is represented by G, the end point of the line adjacent to which there is a *, is not a carbon atom or a CH₂ group but rather a component of the bond to the atom to which G is attached.

In its broadest form, group G having the formula 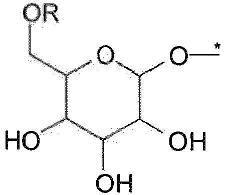 may be also denominated hexose derivative including allose, altrose, glucose, mannose gulose, idose, galactose or talose derivatives having residue R, respectively. The hexose derivatives are preferably D-isomers. The most preferred hexose derivative is a beta-D-galactopyranoside.

R is selected from H, substituted or unsubstituted C₁ to C₅ alkyl, substituted or unsubstituted C₁ to C₁₀ cycloalkyl, or substituted or unsubstituted C₁ to C₁₀ heterocycloalkyl, as indicated above. In case R is substituted or unsubstituted C₁ to C₅ alkyl, preferably unsubstituted C₁ to C₅ alkyl, more preferably methyl, prolonged retention time of the present compound in comparison to R = H may be expected due to an impeded enzymatic cleavage. Said compounds may be therefore present in higher levels within senescent cells.

In formulae, the group which is represented by S, the end point of the line adjacent to which there is a * or #, is not a carbon atom or a CH₂ group but rather a component of the bond to the atom to which S is attached.

In formulae, the group which is represented by L, the end point of the line adjacent to which there is a #, is not a carbon atom or a CH₂ group but rather a component of the bond to the atom to which L is attached. Residue L may be either Z, wherein Z is a radioactive detectable label, a radioactive therapeutic residue, a chelator coordinating a radioactive detectable label or a chelator coordinating a radioactive therapeutic residue. In said case, Z is directly attached to S. Alternatively, residue Z attached via a spacer to S. In this case the spacer has the formula

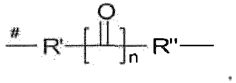

In the context of the present invention, the expression "have/contain" or "having/containing" designates an open enumeration and does not exclude other components apart from the expressly named components.

In the context of the present invention, the expression "consists of" or "consisting of" designates a closed enumeration and excludes any other components apart from the expressly named components.

In the context of the present invention, the expression "essentially consists of" or "essentially consisting of" designates a partially closed enumeration and designates preparations which apart from the named components only have such further components as do not materially alter the character of the preparation according to the invention.

When in the context of the present invention a preparation is described with the use of the expression "have" or "having", this expressly includes preparations which consist of said components or essentially consist of said components.

Radiology as used herein refers to any kind of apparatus or device, capable of producing a visual signal or an image upon detecting the present residue Z, i.e. a radioactive detectable label, a radioactive therapeutic residue, a chelator coordinating a radioactive detectable label or a chelator coordinating a radioactive therapeutic residue. Preferably the apparatus permits localization of residue Z within a mammal. Non limiting examples of radiology include positron-emissions-tomography (PET), which produces a three-dimensional image or map of functional processes in the body. The system detects pairs of gamma rays emitted indirectly by a positron-emitting radioisotope, which is introduced into the body on a metabolically active molecule, i.e. a substrate to beta-galactosidase. Images of metabolic activity in space are then reconstructed by computer analysis, which may be supported by a CT X-ray scan performed on the patient in the same session, preferably at the same time, and in the same device in order to obtain a three dimensional image enabling localization of tissue in which the prodrug is enriched. In positron emission a proton is converted via the weak force to a neutron, a positron and a neutrino. Isotopes, which undergo this so called beta plus decay, thereby emit positrons. Suitable positron-emitting radionuclides for this purpose include ¹¹C, ⁴⁰K, ¹³N, ¹⁵O, ¹⁸F, ⁷⁵Br, ⁷⁶Br, ⁸⁹Zr, ⁸²Rb, ⁶⁸Ga, ⁶²Cu and ⁶⁴Cu, of which ¹¹C and ¹⁸F are preferred. Other useful radionuclides include ¹²³I, ¹²⁴I, ¹²⁵I, ¹³¹I, ²¹⁰At, ²¹¹At and ¹¹¹In. The present compounds may be also labeled with technetium and rhenium isotopes using known chelating complexes. Methods for the generation of radionuclides as well as radio labeling of compounds are well known to the skilled person. US 2007/0273308 and WO 2007/122488 pertain e.g. to the production of radionuclides. Radiolabeling is for example outlined in WO 2007/148089 and WO 2007/148083.

PET is preferably coupled with a computer tomography (CT). Such PET/CT devices enable quantitative detection and allocation of the signals detected to particular tissues, i.e. a localization of the radionuclides employed and hence of the prodrugs attached thereto. Function and operation of PET/CT as well as devices are well known to the skilled person. Other suitable techniques comprise single photon emission computed tomography (SPECT) and techniques based on nuclear magnetic resonance (NMR) wherein the quantum mechanical magnetic properties of an atom's nucleus is detected.

The radiology device employed is preferably a PET device, more preferably a PET/CT device or a PET/MR device.

In the Figures
Fig. 1 shows a motive explaining functioning of a radio-labelled hexose substrate;
Fig. 2 shows the tracer 9 as a substrate of commercially available beta-galactosidase;
Fig. 3 shows stability of the tracer 9 under in-vivo conditions;
Fig. 4 shows *in vitro* validation of tracer in HCT116 and Hras cells;
Fig. 5 shows uptake of tracer 9 in senescent versus control HCT116 tumors;
Fig. 6 shows tracer 9 uptake in senescent versus control Hras tumors; and
Fig. 7 shows immunohistochemical staining from (a) HCT116 and (b) Hras driven tumor models.

G is 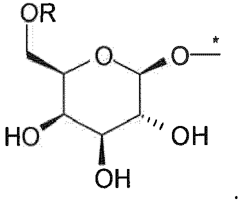 R is H, thereby defining a beta-D-galactose residue, in particular a beta-D-galactopyranoside, which is the best possible sugar moiety for cleavage by beta-galactosidase.

R is H, S is selected from the group consisting of 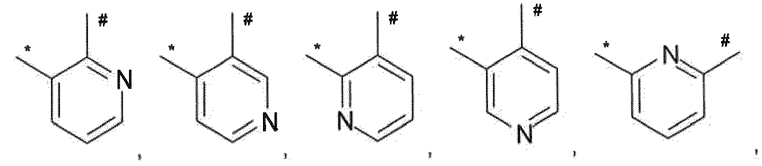 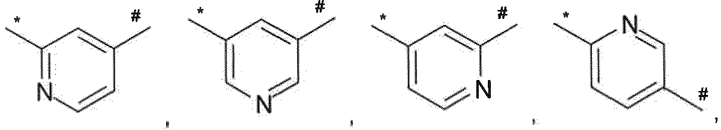 and 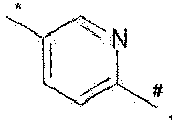 In said compounds, L is 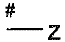 as indiacted above.
Z is preferably ¹⁸F.

R is H, S is selected from the group consisting of 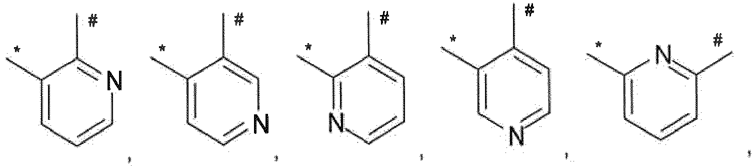 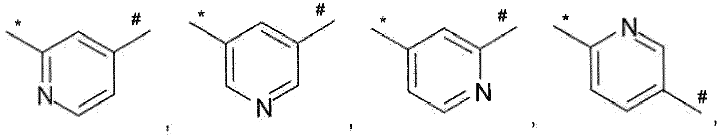 and 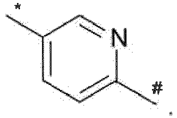 In said compounds, G is 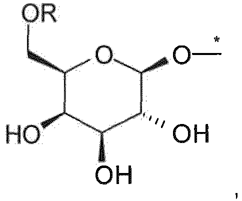 wherein R is H.
L is 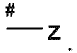 More preferably, Z is ¹⁸F.

According to a preferred embodiment of the present invention the compound is

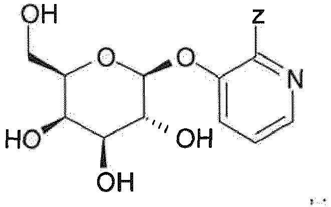

According to another preferred embodiment of the present invention the radioactive detectable label is selected from the group consisting of ¹¹C, ¹⁸F, ⁶⁸Ga, ⁶⁴Cu, and ¹²⁴I, preferably ¹⁸F.

According to a preferred embodiment of the present invention the present compound is for use in surgery. The present compound is preferably employed in combination with at least one inert, non-toxic, pharmaceutically suitable excipient.

According to a preferred embodiment of the present invention the present compound is for use as a medicament. The present compound is preferably employed in combination with at least one inert, non-toxic, pharmaceutically suitable excipient.

According to another preferred embodiment of the present invention the present compound for use in a method for detecting cell senescence.

According to another preferred embodiment of the present invention the present compound is for use in a method of determining the efficiency of cancer treatment.

The cancer to be diagnosed and/or treated by the present compounds may be selected from prostate carcinoma, colorectal carcinoma, breast cancer, lung tumors, tumors of the male or female genitourinary system, malign melanoma, cervix and throat tumor /cervical tumor, malign lymphoma, neoplasia of the hematopoietic system and musculoskeletal tumors.

According to still another preferred embodiment of the present invention the method for detecting cell senescence comprises contacting cells with the present compound, the method is performed *in vitro.*

According to still another preferred embodiment of the present invention the method for determining the efficiency of cancer treatment comprises contacting cells with the present compound, the method is performed *in vitro.*

The above methods can be performed both *in vivo,* e.g. in a human patient for monitoring the efficiency of cancer treatment, or *in vitro,* e.g. for screening for new medicaments.

The present compound will preferably be administered parenterally.

For this administration route, the present compounds may be administered in suitable administration forms. Parenteral administration can take place with avoiding of an absorption step (e.g. intravenous, intraarterial, intracardiac, intraspinal or intralumbar) or with inclusion of an absorption step (e.g. intramuscular, subcutaneous, intracutaneous, percutaneous or intraperitoneal). Administration forms suitable for parenteral administration are, inter alia, preparations for injection and infusion in the form of solutions, suspensions, emulsions, lyophilisates or sterile powders.

The present compounds may be converted into the stated administration forms. This can take place in a manner known per se by mixing with inert, non-toxic, pharmaceutically acceptable excipients. These excipients include inter alia carriers (for example microcrystalline cellulose, lactose, mannitol), solvents (e.g. liquid polyethylene glycols), emulsifiers and dispersants or wetting agents (for example sodium dodecyl sulfate, polyoxysorbitan oleate), binders (for example polyvinylpyrrolidone), synthetic and natural polymers (for example albumin), stabilizers (e.g. antioxidants such as, for example, ascorbic acid), colors (e.g. inorganic pigments such as, for example, iron oxides) and taste and/or odor corrigents.

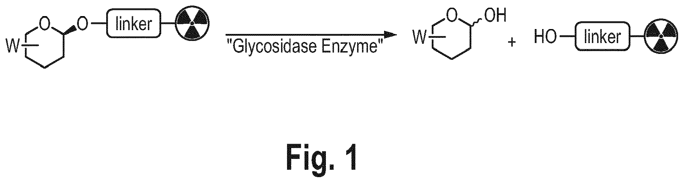

The principle underlying the present invention is generally shown in Fig. 1. A radiolabelled glycosidase substrate, i.e. a compound of the present invention, is converted by a glycosidase, in particular beta-galactosidase, to the corresponding sugar and the radiolabelled alcohol. The present compound is shown with residue W indicative of the moieties forming a hexose derivative, such as a galactose derivative, preferably beta-D-galactose. The glycosidase, particularly the beta-galactosidase, is overexpressed and accumulated in senescent cells. The radiolabelled alcohol in turn is accumulated in acidic lysosomes and may be detected by a radiology device, such as a PET/CT device.

The compounds of the present invention may be generally prepared by the route indicated in reaction scheme I.

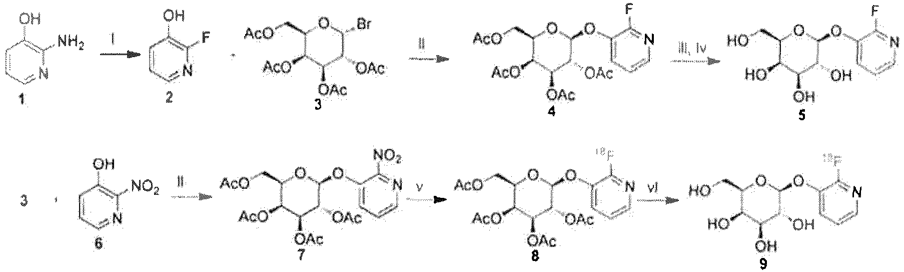

In the upper row of reaction scheme I, generation of compound 5 is shown. Compound 5 does not exhibit a radioactive label, i.e. F is ¹⁹F. Reaction I is conducted with HBF₄, NaNO₂ at a temperature of 0°C. The yield of compound 2 is 63% w/w. Reaction II is conducted with Ag₂CO₃ and at a temperature of 0°C. The yield of compound 4 is between 40 to 60% w/w. Reaction III is conducted with NaOMe and MeOH at a temperature in the range from 20 to 25°C followed by reaction IV in presence of amberlite IR₁₂₀.

In the lower row of reaction scheme I, generation of compound 9 (tracer 9), a compound according to the present invention, is shown. Reaction II is conducted with Ag₂CO₃ at a temperature of 0°C. Reaction VI is conducted with Ag₂CO₃ at a temperature of 0°C. Reaction V is conducted with ¹⁸F⁻ and DMSO at a temperature of 150°C for 5 min. Reaction VI is conducted with NaOMe/ MeOH, or MeOH, Et₃N, H₂O (10:1 :1)

The compounds of the present invention show a valuable range of pharmalogical effects which could not have been predicted. They are capable of marking senescent cells *in vitro* and *in vivo.* Particularly, *in vivo* marking is made to such an extent that senescent cells may be unambiguously identified in course of a surgical procedure which in turn allows targeted elimination of such cells, particularly cancer cells.

It is to be understood that the above description is intended to be illustrative only and not restrictive. Many embodiments will be apparent to those skilled in the art upon reviewing the above description. By way of example, the invention has been described preliminarily with reference to synthesis as well as diagnosis of tracer 9. It should be clear that all kinds of suitable detectable labels and therapeutic residues may be synthesized and attached to the present compounds. The scope of the invention should, therefore, be determined not with reference to the above description, but should instead be determined with reference to the appended claims, along with the full scope of equivalents to which such claims are entitled.

The percentage data in the following tests and examples are, unless indicated otherwise, percentages by weight; parts are parts by weight. Solvent ratios, dilution ratios and concentration data of liquid/liquid solutions are based in each case on volume. The statement "w/v" means "weight/volume". Thus, for example, "10% w/v" means: 100 ml of solution or suspension contain 10 g of substance.

### Examples:

### General

Optimized radio synthesis utilizes a TRACERlab FX N Pro (GE). ¹⁸F is produced as hydrofluoric acid (HF) using a PETtrace cyclotron (GE). ¹⁸F labelling relied on the nucleophilic substitution of an aromatic nitro group. The synthetic route is described in reaction scheme I.

The following subcutaneous animal models are used for tracer evaluation.

A xenograft model of colorectal cancer cell line (HCT116) with therapy induced senescence, doxorubicin is used as a chemotherapeutic. Senescence is induced in HCT116 xenograft tumors by intravenous (i.v.) administration of doxorubicin (10 mg/kg). Through tail vain of the mice, 5 days later dynamic PET/MRT scans (1 h) with compound 9 are performed.

As the second model, transgenic liver progenitor cell line (Hras) was used for subcutaneous allografts in nude mice. The cell line expresses Hras^{G12V} and p53shRNA under doxycycline (doxy) conditions. After the removal of doxycycline, the p53 mRNA can be restored and translated to p53 protein. The high amount of p53 triggers senescence. The animals bearing Hras tumors received doxy-water (0,2 mg/ml) for tumor development and 14 days, after removal of doxy-water, the PET/MRT scans are performed with compound 9.

### Example 1

Example 1 discloses synthesis of compounds according to reaction scheme I.

### Synthesis of Compound 4

(2R,3S, 4S, 5R,6S)-2-(acetoxymethyl)-6-((2-fluoropyridin-3-yl)oxy)tetrahydro-2H-pyran-3,4,5-triyl triacetate, **4**: A solution comprising 2,3,4,6-Tetra-O-acetyl-α-D-galactopyranosyl bromide **3** (1.0 g, 2.4 mmol), Ag₂CO₃ (0.74 g, 2.7 mmol), MS-4Å (5 g) and 2-fluoro-pyridin-3-ol **5** (0.33 g, 2.9 mmol) in anhydrous DCM (15 mL) is stirred overnight, in the dark, at room temperature, under an argon atmosphere. TLC analysis indicated the complete consumption of the starting bromide and the formation of a new nonpolar product. The solution is filtered through a celite, which was rinsed with DCM (3 x 10 mL). After removing the solvent under reduced pressure, the crude residue is purified directly using silica gel column chromatography, using an increasing gradient of EtOAc in PE. The product afforded is a colorless solid (0.51 g, 51 %).

TLC: R*_{f}*= 0.5 (EtOAc: PE = 1: 1); ¹H NMR (600 MHz, Chloroform-*d*) δ 7.94 (d, *J* = 4.6 Hz, 1H, ArH), 7.58 (t, *J* = 7.8 Hz, 1H, ArH), 7.13 (dd, *J* = 7.8, 4.8 Hz, 1H, ArH), 5.50 (dd, *J* = 10.5, 7.9 Hz, 1H), 5.45 (d, *J* = 3.4 Hz, 1H), 5.10 (dd, *J* = 10.5, 3.4 Hz, 1H), 4.96 (d, *J* = 7.9 Hz, 1H), 4.22 (dd, *J* = 11.4, 6.8 Hz, 1H), 4.15 (dd, *J* = 11.4, 6.3 Hz, 1H), 4.01 (td, *J* = 6.8, 1.1 Hz, 1H), 2.19 (s, 3H, OAc), 2.11 (s, 3H, OAc), 2.04 (s, 3H, OAc), 2.02 (s, 3H, OAc); ¹³C NMR (151 MHz, CDCl₃) δ 170.26 (CO_{quart}), 170.13 (CO_{quart}), 170.04 (CO_{quart}), 169.44 (CO_{quart}), 154.75 (d, *J* = 239.9 Hz), 141.59 (d, *J* = 13.4 Hz, ArC), 139.49 (d, *J* = 25.5 Hz, ArC), 130.22 (d, *J* = 3.5 Hz, ArC), 121.85 (d, *J* = 4.3 Hz, ArC), 121.23 (CH), 101.12 (CH), 71.38 (CH), 70.5 (CH), 68.29 (CH), 66.70 (CH), 61.17 (CH₂), 21.03 (OAc), 20.63 (OAc), 20.61 (OAc), 20.55 (OAc).

### Synthesis of Compound 5

(2S,3R,4S,5R,6R)-2-((2-fluoropyridin-3-yl)oxy)-6-(hydroxyl-methyl)tetrahydro-2H-pyran-3,4,5-triol, **5:** Catalytic sodium methoxide is added to a solution of **4** (0.5 g, 1.1 mmol) in dry methanol (5 mL). The solution is stirred at room temperature until TLC analysis revealed that the hydrolysis of the acetylated sugar is complete. Acetic acid (0.2 mL) is added, after which the solvents are removed under reduced pressure. The remaining solid was of high purity, but subsequent recrystallization from methanol afforded 6 as a highly pure crystalline solid (225 mg, 73 %).

TLC: R*_{f}* = 0.25 (EtOAc: MeOH = 9: 1); ¹H NMR (600 MHz, Deuterium Oxide) δ 7.79 (dd, *J* = 8.1, 5.0 Hz, 1H, ArH), 7.72 (t, *J* = 8.9, 8.1 Hz, 1H, ArH), 7.26 (dd, *J* = 8.0, 5.0 Hz, 1H, ArH), 5.06 (d, *J* = 7.8 Hz, 1H), 3.95 (dd, *J* = 3.4, 1.8 Hz, 1H), 3.84 - 3.78 (m, 2H), 3.75 - 3.70 (m, 3H), 1.85 (dd, *J* = 2.3, 1.1 Hz, 1H); ¹³C NMR (151 MHz, Deuterium Oxide) δ 153.70 (d, *J* = 238.5 Hz, CF), 141.49, 139.72 (d, *J* = 23.9 Hz, ArC), 139.29 (d, *J* = 11.6 Hz, ArC), 128.11 (d, *J* = 3.7 Hz, ArC), 122.75 (d, *J* = 4.2 Hz, ArC), 101.24 (CH), 75.68 (CH), 72.41 (CH), 70.31 (CH), 68.36 (CH), 60.68 (CH₂); HRMS (ESI): [M + Na ]⁺ (ₜₕₑₒᵣ.) = 298,06974, measured = 298,06998

### Synthesis of compound 7

(2*R*,3*S*,4*S*,5*R*,6*S*)-2-(acetoxymethyl)-6-((2-nitropyridin-3-yl)oxy)tetrahydro-2*H*-pyran-3,4,5-triyl triacetate, **7:** A solution comprising 2,3,4,6-Tetra-O-acetyl-α-D-galactopyranosyl bromide **3** (1.0 g, 2.4 mmol), Ag₂CO₃ (1.3 g, 4.8 mmol), MS-4Å (5 g) and 2-nitro-pyridin-3-ol **6** (1.4 g, 9.7 mmol) in anhydrous DCM (10 mL) is stirred over night, in the dark, at room temperature, under an argon atmosphere. TLC analysis indicates the complete consumption of the starting bromide and the formation of a new nonpolar product. The solution is filtered through a celite, which is rinsed with DCM (3 x 10 mL). After removing the solvent under reduced pressure, the crude residue is purified directly using silica gel column chromatography, using an increasing gradient of EtOAc in PE. The product **7** afforded is a colorless solid (0.7 g, 63 %).

TLC: R*_{f}* = 0.65 (EtOAc: PE = 4: 1); ¹H NMR (600 MHz, Chloroform-*d*) δ 8.26 (dd, *J* = 4.6, 1.4 Hz, 1H, ArH), 7.82 (dd, *J* = 8.4, 1.4 Hz, 1H, ArH), 7.54 (dd, *J* = 8.4, 4.6 Hz, 1H, ArH), 5.51 (dd, *J* = 10.5, 7.9 Hz, 1H), 5.47 (dd, *J* = 3.4, 1.2 Hz, 1H), 5.10 (dd, *J* = 10.5, 3.4 Hz, 1H), 5.06 (d, *J* = 7.9 Hz, 1H), 4.25 (dd, *J* = 11.4, 6.1 Hz, 1H), 4.16 (dd, *J* = 11.4, 6.1 Hz, 1H), 4.06 (ddd, *J* = 6.1, 6.1, 1.3 Hz, 1H), 2.19 (s, 3H, OAc), 2.14 (s, 3H, OAc), 2.06 (s, 3H, OAc), 2.01 (s, 3H, OAc); ¹³C NMR (151 MHz, CDCl₃) δ 170.21 (CO_{quart}), 170.04 (CO_{quart}), 170.01 (CO_{quart}), 169.32 (CO_{quart}), 150.42 (ArC_{quart}), 144.25 (ArC_{quart}), 142.91 (ArC), 129.86 (ArC), 128.29 (ArC), 100.94 (CH), 71.65 (CH), 70.31 (CH), 67.64 (CH), 66.58 (CH), 61.26 (CH₂), 20.62 (OAc), 20.60 (OAc), 20.56 (OAc), 20.51 (OAc); HRMS (ESI): [M + Na]⁺ _{(theor.)} = 493,10649 measured = 493,10686.

### Synthesis of compounds 8 and 9

Radiolabeling of **7** proceeds readily in DMSO at 150°C. Azeotropically dried [K.2.2.2]^{+ 18}F⁻ fluoride complex is used, producing the acetylated ¹⁸F-labelled intermediate, which is purified using semi-preperative HPLC. The tracer was concentrated by trapping onto a C₁₈ -SPE cartridge, deprotected with NaOH (185 µL, 2.0 M) to produce tracer 9 and eluted with 10% ethanol in water (3 mL). It is appropriately formulated with HCl (195 µL, 2.0 M), NaHCO₃ (500 µL, 1.0 M) and water (5 mL) to ensure subisotonic sodium concentration (0.1 M) and a final pH (7.5) that is biologically tolerated. The tracer is produced with a radiochemical purity of >98%, the decay corrected yield was 18.6+/- 2.5% (n=10) with a molar radioactivity of 18.8±3.5 GBq*µmole⁻¹ (n=5).

### Example 2

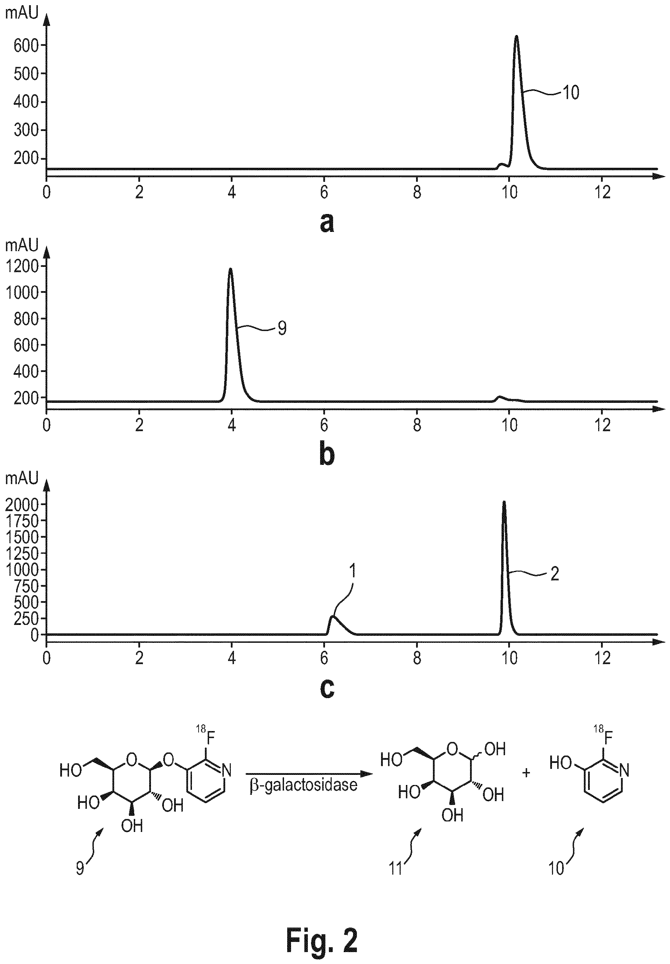

To assess whether tracer 9 is a substrate of beta-galactosidase, it is incubated at 30°C for 30 minutes in citrate buffer of pH 5.5 containing commercially available beta-galactosidase (Sigma). The reaction is stopped by adding 2 volume equivalents of acetonitrile, followed by 5 minutes of centrifugation. The supernatant is examined directly using radio-HPLC. The reaction produces the radioactive metabolite with the same retention time as the non-radioactive 2-fluoropyridinol. The expected radioactive metabolite is compared with the authentic non-radioactive standard (Fig. 2). In particular, compound 10 is radioactive 2-fluoropyridinol (Fig. 2a), compound 9 is tracer 9 (Fig. 2b), and compounds 1 and 2 are other compounds of reaction scheme I (Fig. 2c). Compound 11 is galactose.

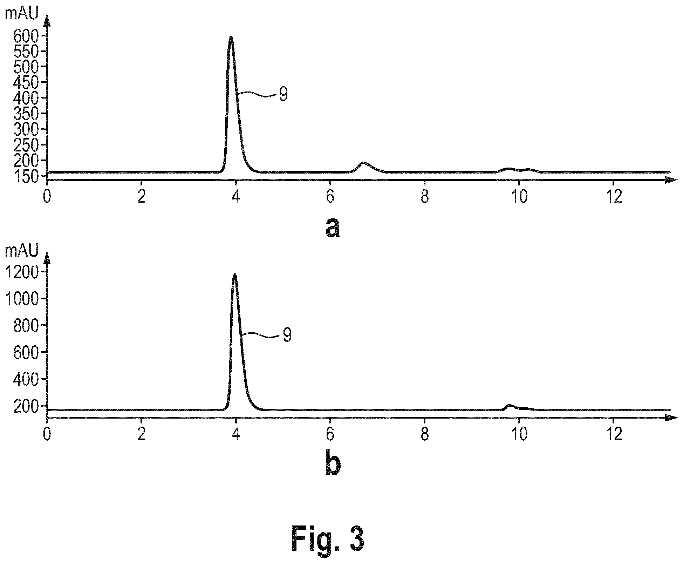

The tracer 9 is incubated at 37°C in mouse serum to asses its stability. After 1 hour, 2 volume equivalents of acetonitrile are added and the mixture is centrifuged for 5 minutes. The supernatant is examined directly using radio-HPLC. Serum stability of tracer 9 after 1 h (Fig. 3a) vs tracer 9 (Fig. 3b) is derivable from Fig. 3. The experiment reveals a few minor radioactive metabolites, but tracer's integrity remains largely intact.

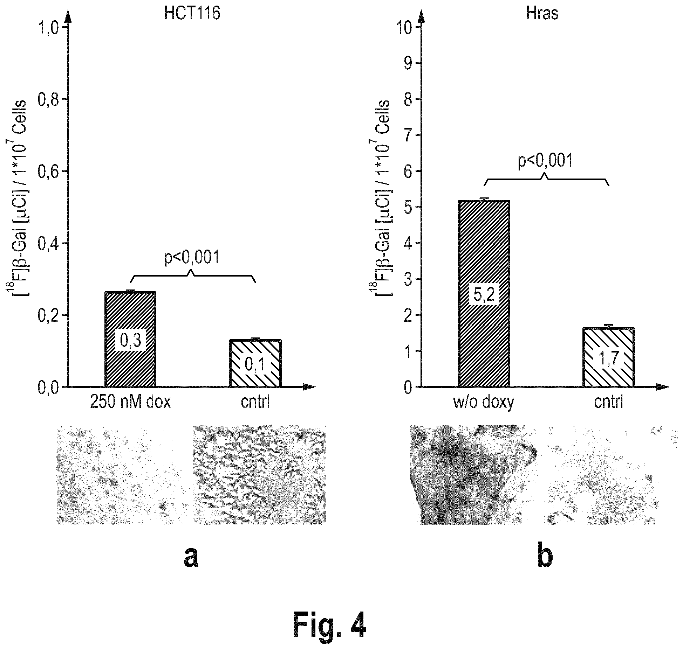

In-vitro evaluation of compound 9 is shown in Fig. 4. HCT116 (Fig. 4a) and Hras cells (Fig. 4b) are treated with 100-200 µCi of tracer and incubated for 50 minutes. The cells are measured in a gamma-counter, with the activity normalized to the number of cells used. In both cases, the senescent cells show significantly higher uptake compared to the controls.

### Example 3

The PET-tracer is tested in two different models *in vitro.* In HCT116 cells senescence is induced by overnight incubation with doxorubicin, followed by 4 days of culture under normal conditions. Senescence in a HRas driven liver progenitor cell line with a doxycycline regulatable p53-specific shRNA. After induction of senescence, both cell lines are incubated with 3.7 - 7.4 MBq of tracer for 50 minutes. The cells are then washed, counted and measured in a gamma counter. The activity taken up by the cells is analyzed and normalized to a million cells.

*In vivo* tests are performed in nude mice bearing s.c. HCT116 or HRas driven liver progenitor cells. Mice bearing HCT116 tumors are treated with doxorubicin (10 mg/Kg bodyweight) and imaged 4 days after the onset of senescence. Mice bearing Hras driven tumor are supplied with doxycycline in their drinking water, which has been later removed to induce senescence. After induction of senescence, the tracer is administered i.v. and PET scans are performed. The %ID/cc and tumor-to-muscle ratios are determined through quantitative analysis of the PET data, permitting evaluation of the tracers.

*In vitro* senescent HCT116 cells show an uptake of 11 kBq/1 mio cells, while the uptake in non-senescent control cells is only 4 kBq/1 mio cells. In the HRas driven liver progenitor model senescent cells show an uptake of 192 kBq/1 mio cells and is significantly increased in comparison to non-senescent cells (63 kBq/1 mio cells).

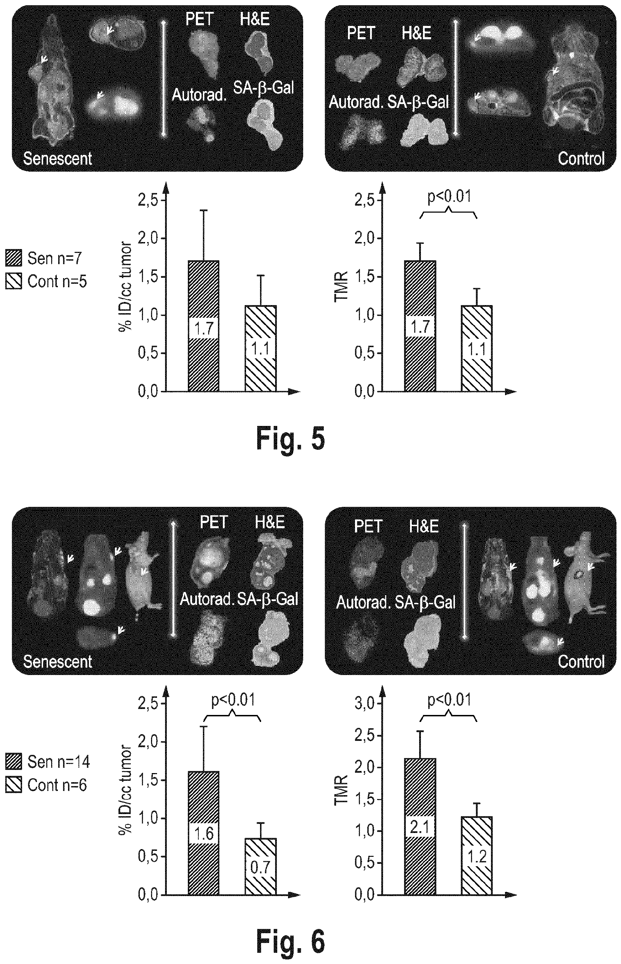

The *in vivo* tracer uptake in senescent and non-senescent HCT116 tumors (Fig. 5) is 1.7+/-0.7 (n=7) vs 1.1+/-0.4 (n=5) %ID/cc respectively; the respective TMR values are 1.7 and 1.1. In particular, Fig. 5 indicates that tracer 9 shows higher uptake in senescent vs control HCT116 tumors. Both %ID/cc and TMR are higher in senescent vs control tumors. The excised tumors are subjected to *ex vivo* analyses. Autoradiography confirmed findings, showing higher tracer uptake in the senescent tumor section vs control. Senescent tumor slices also showed higher X-Gal staining than control sections.

In the HRas driven model (Fig. 6), the tracer uptake in senescent tumors is significantly increased to that of control tumors (1.5+/-0.3 (n=16) vs 0.9+/-0.3 (n=11) %ID/cc); the TMR is subject to a similar trend, with respective values of 2.1 and 1.2. Fig. 6 indicates that tracer 9 shows higher uptake in senescent vs control HRas tumors. Both %ID/cc and TMR are higher in senescent vs control tumors. The excised tumors are subjected to ex *vivo* analyses. Autoradiography confirmed findings, showing higher tracer uptake in the senescent tumor section vs control. Senescent tumor slices also show higher X-Gal staining than control sections. The nonsenescent tumor expresses GFP, which can be visualized with optical imaging.

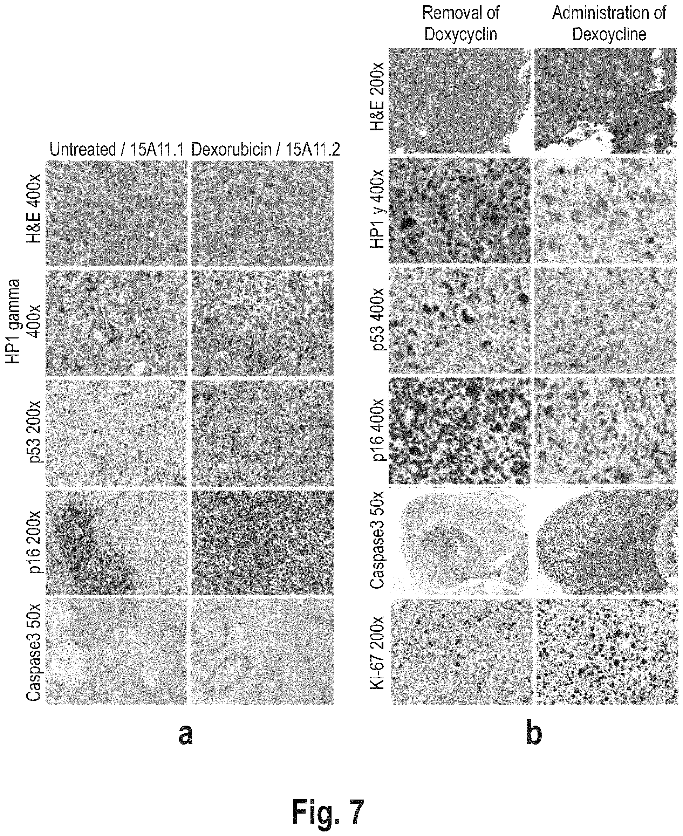

Induction of senescence is confirmed by ex *vivo* beta-gal staining and immunohistology of Ki67, Caspase3, HP1γ, p53 and p16 (Fig. 7). Fig. 7 shows immunohistochemical staining from HCT116 (Fig. 7a) and HRas (Fig. 7b) driven tumor models. In both cases, immunohistochemical analysis was performed and reveals an increase of the expected senescence markers HP1γ and p53. The Hras driven tumors also show high expression of ki67. The low abundance of caspase-3 in both tumor models suggests that apoptosis is not a key contributor to the tumors status and confirms assertion of senescence.

## Claims

1. A compound of the formula:
G - S - L,
wherein
G is 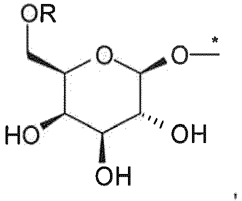 wherein R is H,
and wherein * represents the binding site between G and S,
S is selected from the group consisting of 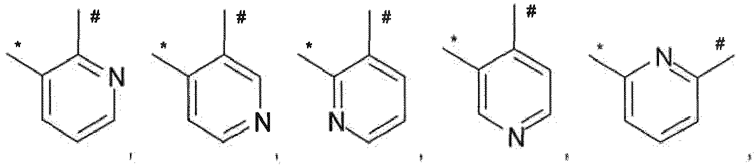 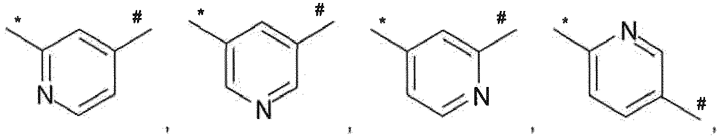 and 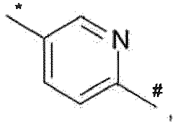 wherein # represents the binding site between S and L, and
L is 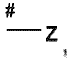
wherein Z is a radioactive detectable label, a radioactive therapeutic residue; or a salt thereof,
wherein the radioactive detectable label is selected from the group consisting of ¹¹C, ⁴⁰K, ¹³N, ¹⁵O, ¹⁸F , ⁷⁵Br, ⁷⁶Br, ⁸²Rb, ⁶⁸Ga, ⁶⁴Cu, ⁶²Cu, ⁸⁹Zr, ¹²³I, ¹²⁴I, ¹²⁵I, ¹³¹I, ²¹⁰At, ²¹¹At and ¹¹¹In,
wherein the radioactive therapeutic residue is selected from the group consisting of ³²P, ⁶⁰Co, ⁶⁴Cu, ⁸⁹Sr, ⁹⁰Y, ¹⁷⁷Lu, ¹⁸⁶Re and ¹⁵³Sm.

2. The compound according to claim 1, wherein the compound is

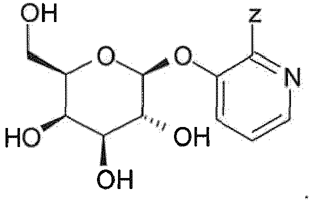

3. The compound according to any of the preceding claims, wherein the radioactive detectable label is selected from the group consisting of ¹¹C, ¹⁸F, ⁶⁸Ga, ⁶⁴Cu, and ¹²⁴I, preferably ¹⁸F.

4. The compound according to claim 1, for use in surgery, wherein Z is the radioactive detectable label; or the salt thereof.

5. The compound according to claim 4 for use as a medicament, wherein Z is the radioactive therapeutic residue; or the salt thereof.

6. The compound according to claims 1 to 3 for use in a method for detecting cell senescence.

7. The compound according to any one of claims 1 to 3 for use in a method of determining the efficiency of cancer treatment.

8. Method for detecting cell senescence comprising contacting cells with a compound according to any one of claims 1 to 3, **characterised in that** the method is performed *in vitro.*

9. Method for determining the efficiency of cancer treatment comprising contacting cells with a compound according to any one of claims 1 to 3, **characterised in that** the method is performed *in vitro.*

## Patentansprüche

1. Verbindung der Formel
G - S - L,
in welcher
G 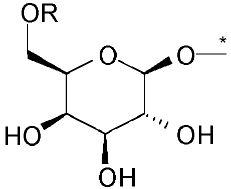 ist,
wobei R H ist,
und wobei * die Bindungsstelle zwischen G und S darstellt,
S ausgewählt ist aus der Gruppe bestehend aus 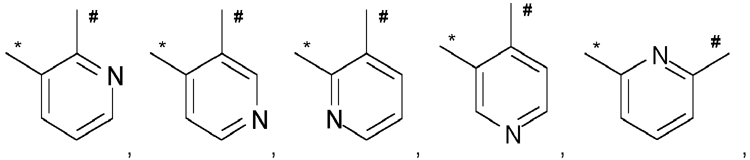 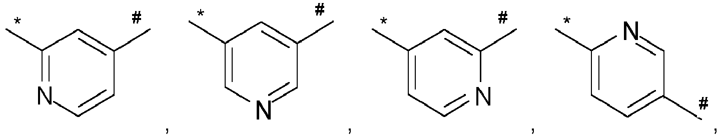 und 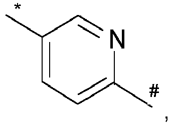 wobei # die Bindungsstelle zwischen S und L darstellt, und
L 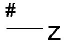 ist,
wobei Z ein radioaktiver detektierbarer Marker, ein radioaktiver therapeutischer Rest; oder ein Salz davon ist,
wobei der radioaktive detektierbare Marker ausgewählt ist aus der Gruppe bestehend aus ¹¹C, ⁴⁰K, ¹³N, ¹⁵O, ¹⁸F, ⁷⁵Br, ⁷⁶Br, ⁸²Rb, ⁶⁸Ga, ⁶⁴Cu, ⁶²Cu, ⁸⁹Zr, ¹²³I, ¹²⁴I, ¹²⁵I, ¹³¹I, ²¹⁰At, ²¹¹At und ¹¹¹In,
wobei der radioaktive therapeutische Rest ausgewählt ist aus der Gruppe bestehend aus ³²P, ⁶⁰Co, ⁶⁴Cu, ⁸⁹Sr, ⁹⁰Y, ¹⁷⁷Lu , ¹⁸⁶Re und ¹⁵³Sm.

2. Verbindung nach Anspruch 1, wobei die Verbindung die folgende ist:

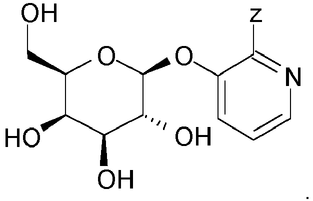

3. Verbindung nach einem der vorhergehenden Ansprüche, wobei der radioaktive detektierbare Marker ausgewählt ist aus der Gruppe bestehend aus ¹¹C, ¹⁸F, ⁶⁸Ga, ⁶⁴Cu, und ¹²⁴I, vorzugsweise ¹⁸F.

4. Verbindung nach Anspruch 1 zur Verwendung in der Chirurgie, wobei Z der radioaktive detektierbare Marker ist; oder das Salz davon.

5. Verbindung nach Anspruch 4 zur Verwendung als Arzneimittel, wobei Z der radioaktive therapeutische Rest ist, oder das Salz davon.

6. Verbindung nach Anspruch 1 bis 3 zur Verwendung in einem Verfahren zur Detektion von Zellseneszenz.

7. Verbindung nach einem der Ansprüche 1 bis 3 zur Verwendung in einem Verfahren zur Bestimmung der Wirksamkeit einer Krebsbehandlung.

8. Verfahren zur Detektion von Zellseneszenz, das das Inkontaktbringen von Zellen mit einer Verbindung gemäß einem der Ansprüche 1 bis 3 aufweist, **dadurch gekennzeichnet, dass** das Verfahren *in vitro* ausgeführt wird.

9. Verfahren zur Bestimmung der Wirksamkeit einer Krebsbehandlung, das das Inkontaktbringen von Zellen mit einer Verbindung gemäß einem der Ansprüche 1 bis 3 aufweist, **dadurch gekennzeichnet, dass** das Verfahren *in vitro* ausgeführt wird.

## Revendications

1. Composé de la formule :
G - S - L,
G étant 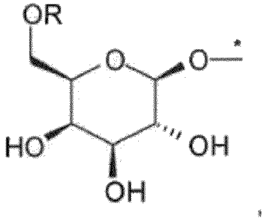
R étant H,
et * représentant le site de liaison entre G et S,
S étant choisi dans le groupe constitué par 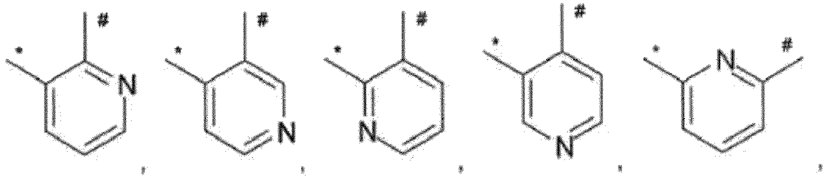 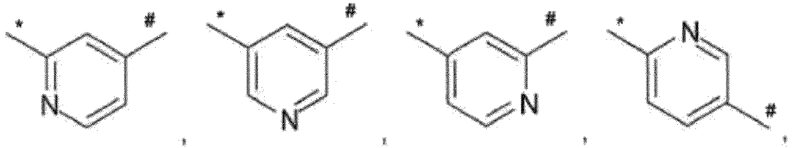 et 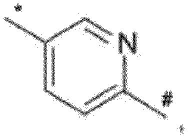 # représentant le site de liaison entre S et L, et
L étant 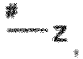
Z étant un marqueur détectable radioactif, un résidu thérapeutique radioactif ; ou un sel correspondant,
le marqueur détectable radioactif étant choisi dans le groupe constitué par ¹¹C, ⁴⁰K, ¹³N, ¹⁵O, ¹⁸F, ⁷⁵Br, ⁷⁶Br, ⁸²Rb, ⁶⁸Ga, ⁶⁴Cu, ⁶²Cu, ⁸⁹Zr, ¹²³I, ¹²⁴I, ¹²⁵I, ¹³¹I, ²¹⁰At, ²¹¹At et ¹¹¹In,
le résidu thérapeutique radioactif étant choisi dans le groupe constitué par ³²P, ⁶⁰Co, ⁶⁴Cu, ⁸⁹Sr, ⁹⁰Y, ¹⁷⁷Lu,¹⁸⁶Re et ¹⁵³Sm.

2. Composé selon la revendication 1, le composé étant

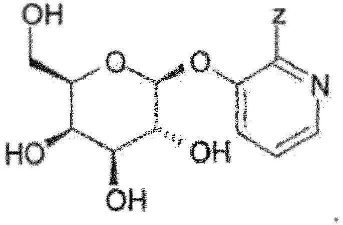

3. Composé selon l'une quelconque des revendications précédentes, le marqueur détectable radioactif étant choisi dans le groupe constitué par ¹¹C, ¹⁸F, ⁶⁸Ga, ⁶⁴Cu, et ¹²⁴I, préférablement ¹⁸F.

4. Composé selon la revendication 1, pour une utilisation en chirurgie, Z étant le marqueur détectable radioactif ; ou le sel correspondant.

5. Composé selon la revendication 4 pour une utilisation en chirurgie, Z étant le résidu thérapeutique radioactif ; ou le sel correspondant.

6. Composé selon les revendications 1 à 3 pour une utilisation dans un procédé pour la détection de sénescence cellulaire.

7. Composé selon l'une quelconque des revendications 1 à 3 pour une utilisation dans un procédé de détermination de l'efficacité d'un traitement d'un cancer.

8. Procédé pour la détection de sénescence cellulaire comprenant la mise en contact de cellules avec un composé selon l'une quelconque des revendications 1 à 3, **caractérisé en ce que** le procédé est réalisé *in vitro.*

9. Procédé pour la détermination de l'efficacité d'un traitement d'un cancer comprenant la mise en contact de cellules avec un composé selon l'une quelconque des revendications 1 à 3, **caractérisé en ce que** le procédé est réalisé *in vitro.*
